# EUROPEAN PATENT APPLICATION

(11) **EP 2 286 669 A1**
(43) Date of publication of application: **23.02.2011**
(21) Application number: 09166700.6
(22) Date of filing: 29.07.2009
(51) Int. Cl.: A23C 9/152, A23L 1/30, A61K 31/7048, A61P 17/00, A61P 19/08, A61K 36/899

(54) **Flavanones-containing food compositions**

(71) Applicant: Nestec S.A., 1800 Vevey (CH)
(72) Inventor: Hoebler, Pascaline, 3286, MUNTELIER (CH)
(74) Representative: Chautard, Cécile

(57) **Abstract**

The present invention relates to food products comprising flavanones. In particular, it relates to food products comprising hesperidin having improved stability. The present invention also concerns processes for the manufacturing of food products comprising said flavanones, especially hesperidin and to the use of the food products in the manufacture of compositions for the improvement of bone and skin health.

## Description

### Field of the present invention

The present invention relates to food products comprising flavanones. In particular, it relates to food products comprising hesperidin and having improved stability. The present invention also concerns processes for the manufacturing of food products comprising said flavanones, especially hesperidin and to the use of the food products in the manufacture of compositions for the improvement of bone and skin health.

### Background of the invention

Hesperidin is a known flavonoid compound which has a number of health benefits associated to its uptake. These benefits include antioxidant properties, antiinflammatory properties, cardio-protective and lipid lowering effect, anti-microbial activity, anti-carcinogenic activity, UV protective activity etc. Medicinal applications of hesperidin are for example disclosed in EP 1 536 806.

Commercially available hesperidin is difficult to incorporate into foods due to its poor water or oil solubility. Due to these difficulties, it has only been possible so far to include hesperidin in products where sedimentation is not an issue, such as food supplements in the form of powders, or chewing gum for example, or in liquid products where the hesperidin concentration is very low.

Such liquid products containing flavanones such as hesperidin are known from prior art. For instance, US 3615717 describes a liquid milk product which comprises hesperidin in an amount of at most 0.04% in order to inhibit staling.

In US 2006/0105089 a refined peel juice comprising at most 70ppm hesperidin is described.

Such low concentrations of flavanones as are described in the liquid products of the prior art do not impact on the issue of storage stability or sedimentation.

A suggested approach to incorporate hesperidin in higher amounts into a liquid product includes wet, dry and jet milling of hesperidin prior to integration in the liquid product. This approach is however very costly and requires investment in a specific processing equipment.

Another approach is to use stabilising agents and/or increase the viscosity of the liquid product to prevent rapid sedimentation of hesperidin. Although this is applicable to liquid products, at high concentration at which it could stabilize hesperidin, it would affect the sensorial properties of the product, e.g. viscosity. This approach is also not easily applicable for the manufacture of powder products to be reconstituted in a liquid.

### Object of the present invention

It is therefore an object of the present invention to improve the stability of liquid food products or reconstituted powders comprising flavanones, especially hesperidin and to provide a wider range of food products comprising said flavanones.

### Summary of the invention

This object is solved by means of the independent claims. The dependent claims further serve to develop the central idea of the invention.

Thus, in a first aspect, the invention relates to a process for the manufacture of a food product comprising flavanones, comprising the step of setting the pH of an aqueous solution of flavanone to a value of 10-14, preferably 12 to 13 prior to mixing the solution with components of the food product.

A food product obtainable by such process constitutes a further aspect of the invention.

The invention further relates to a process for improving the stability of flavanones in a liquid or semi-liquid food product comprising the step of setting the pH of an aqueous solution of flavanone to a value of 10-14, preferably 12 to 13 prior to mixing the solution with components of the food product.

A food product comprising 0.05-5 wt% flavanones, especially hesperidin, wherein said flavanones are obtained by setting the pH of an aqueous solution of flavanone to a value of 10-14, preferably 12 to 13 prior to mixing the solution with components of the food product, also forms part of the present invention.

Finally, the invention also provides for the use of a food product according to any of claims 9 to 13 for the manufacture of a composition for the improvement of bone and skin health.

Finally, a process for producing stabilised flavanones comprising the step of setting the pH of an aqueous solution of flavanone to a value of 10-14, preferably 12 to 13 and the flavanone obtainable by such process are also part of the invention.

### Figures

The present invention is further described hereinafter with reference to some of its embodiments shown in the accompanying drawings in which:
- Fig. 1 is a flow chart illustrating the alkali treatment process according to the invention (TS: total solids)
- Fig. 2.1 and 2.2 show the particle size distribution of commercially available hesperedin compared to hesperidin having undergone the process of the invention respectively.
- Fig. 3 shows the microscopy results of hesperidin as raw material (RM) and after having undergone the process of the invention.
- Fig. 4.1 and 4.2 show the stability results in a reconstituted milk using 1% hesperidin treated according to the present invention (4.2) compared to commercially available hesperidin (4.1) after 5 minutes.
- Figures 5.1, 5.2 and 5.3 show respectively the particle size distribution of hesperidin in water, wherein the hesperidin is commercially available (variant 1), hesperidin wherein the particle size has been reduced by dry milling (variant 2) and hesperidin which has been treated according to a process of the invention (variant 3).
- Figures 6.1 and 6.2 show the pictures of a reconstituted milk comprising hesperidin which is commercially available (variants 1), hesperidin wherein the particle size has been reduced by dry milling (variant 2) and hesperidin which has been treated according to a process of the invention (variant 3) taken after 5 minutes and after 12 hours respectively.

### Detailed description of the invention

The invention is described in the following by reference to hesperidin as a preferred embodiment of the invention. However, the invention equally relates to other flavanones such as eriocitrin, naringin, etc. Preferably, the flavanones are selected from those which are water insoluble. By "water insoluble" is meant that the flavanones do not dissolve completely in water at a room temperature (e.g. 20°C) at a neutral pH (e.g. about 7). Preferably, the flavanones are soluble in alkaline conditions, e.g. at a pH of 10-14.

The present invention relates to a process for the manufacture of a food product comprising hesperidin. The hesperidin obtained by carrying out the process step is stabilised which constitutes an advantage for the food product in terms of storage stability and organoleptic properties. By "stabilised" is meant that, compared to hesperidin which has not undergone the processes of the invention, the hesperidin in the food product does not easily sediment out of solution. The hesperidin in the stabilised form of the invention confers stability upon storage to the product containing it.

In one of its embodiments, the present invention relates to a process for the manufacture of a food product comprising hesperidin. The process comprises the step of setting the pH of an aqueous solution of hesperidin to a value of between 10 and 14, preferably between 12 and 13, more preferably to about 12.

The pH of the aqueous solution is set to these values by adding any food-grade bases or alkali solutions, such as sodium hydroxide, potassium hydroxide, calcium hydroxide etc.

The aqueous solution preferably comprises 0.1-30 wt% hesperidin, preferably 0.1-20 wt% more preferably about 5 wt%.

Once the pH is set to these values, the aqueous solution is mixed with components of the food product. By components of the food product is meant either parts of the food product or the whole food product.

The mixture of hesperidin-containing solution and the food product may be further processed, for example by subjecting the mixture to standard food processing steps. These may include adjusting the pH, heat treatment, pasteurisation, homogenisation, drying, spray-drying, preheating, cooling, filling or any combinations thereof.

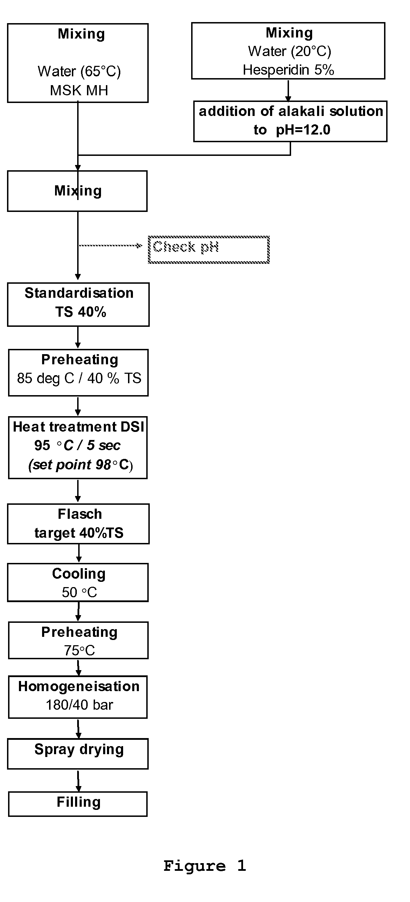

In a preferred embodiment of the invention depicted in figure 1, the food product is skimmed milk powder. Thus, the alkalinised hesperidin-containing aqueous solution is mixed to skimmed milk. The pH is checked and if necessary adjusted to a neutral pH and the mixture is then processed according to standard manufacturing processes for providing a skimmed milk powder. Thus, the mixture may be standardised to a total solids content of 40%. This step may be followed by a pre-heating step at about 85°C, followed by a heat treatment by direct steam injection at 95°C for about 5 seconds. The mixture may then be de-aerated and cooled to 50°C. The mixture may then be preheated again to about 75°C prior to homogenisation in two-stages at 180 bars and 40 bars. Spray-drying may then be carried out according to standard processes.

The resulting product is a skimmed milk powder which may comprise 0.01-5wt%, preferably 0.05-3wt% hesperidin, wherein the particle size of hesperidin is considerably reduced compared to raw hesperidin.

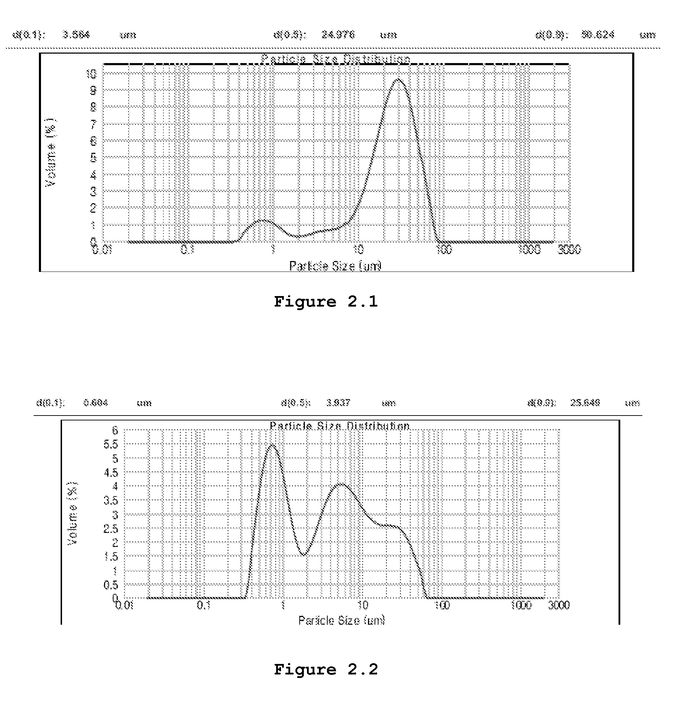

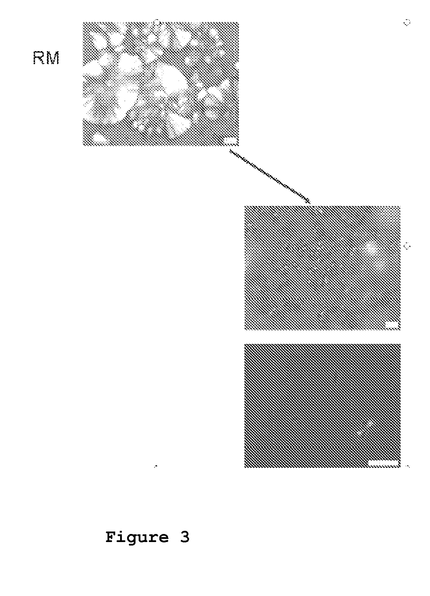

Indeed, it has been found that by the process of the present invention, the particle size distribution of hesperidin is considerably reduced compared to "raw" hesperidin (cf. figs 2.1 and 2.2 and fig.3). Without wishing to be bound to theory, it is thought that this reduction of particle size, along with morphology changes is responsible for conferring to the hesperidin particles more stability in solution.

Upon reconstitution, the skim milk powder provides a skim milk having good organoleptic properties and stability. By stability is meant that no sedimentation or visible changes occur in the food product over a period of time of preferably at least 12 hours.

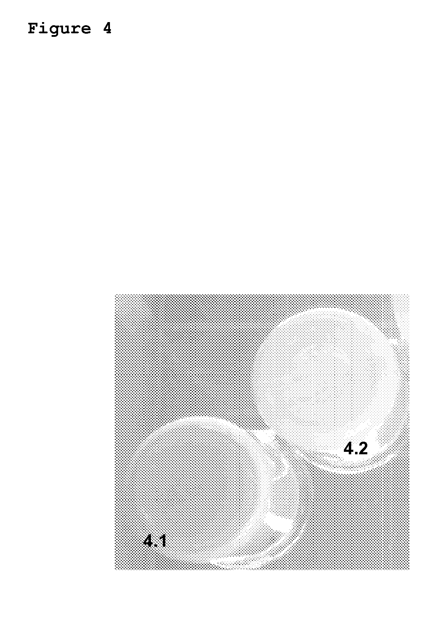

As an illustrative example, figures 4.1 and 4.2 show the difference between the reconstituted powder manufactured by a process of the invention (variant 2) and reconstitution of a powder comprising hesperidin and which has not undergone the process of the invention (variant 1). The pictures clearly show that in sample of variant 1, a brown sediments forms already after 5 minutes after reconstitution, while sample of variant 2 remains stable.

Preferably, the food product which may be liquid or semi-liquid or which may have been reconstituted from the powders of the invention has a total solids content of at least 1%, preferably at least 8%. The amount of total solids can be up to 60%.

Although the above describes in detail a process for making a skimmed milk powder, the process of the invention is not limited thereto. The alkali treatment of hesperidin followed by mixing with food components may therefore be used to manufacture any of skim milk, full milk, fruit juice, sugar solution, sweetened or unsweetened condensed milk, juices, coffee or cereal beverage, chocolate beverage, nutritional oral supplement, soup, shake, or powders such as milk powder, dehydrated soup powder, shake powder, infant formulae etc.

The hesperidin obtainable by the processes of the invention has a smaller size distribution compared to commercially available hesperidin (raw material - RM) as illustrated in figures 2.1 and 2.2..

In addition, the morphology of the hesperidin particles is changed by the process (cf. fig. 3).

Thus, the process of the invention also stabilises hesperidin.

Food products obtainable by the processes of the invention also form part of the present invention. In the most preferred embodiments of the invention, the food product is a milk powder or milk.

The present invention relates, in a further aspect, to food products comprising flavanones, especially hesperidin. Commercially available hesperidin normally has a crystalline form wherein the crystals have a size distribution ranging from 0.1 to 160 microns and with an average of 40% of cumulative distribution of hesperidin particles below 20 microns. The food products of the invention comprise hesperidin, wherein at least 80% of the cumulative distribution of hesperidin particles have a particle size below 20 microns. By particles is meant any physical shape or form which may be crystalline, rod-like shapes, circular or near circular shapes, needle-like shapes, ∞-like shapes, etc.

Figure 3 shows microscopy pictures of hesperidin as a commercially available material in water (RM). It further shows that the hesperidin according to the process of the invention has a much smaller particle size. The microscopy pictures further show that the hesperidin particles having undergone the process of the invention change configuration.

The particle size distribution is observed using a microscopy optic LEICA DMR (mode Differential Interference Contrast and polarisation). The particle size distribution is measured by laser diffraction using a Malvern Mastersizer 2000.

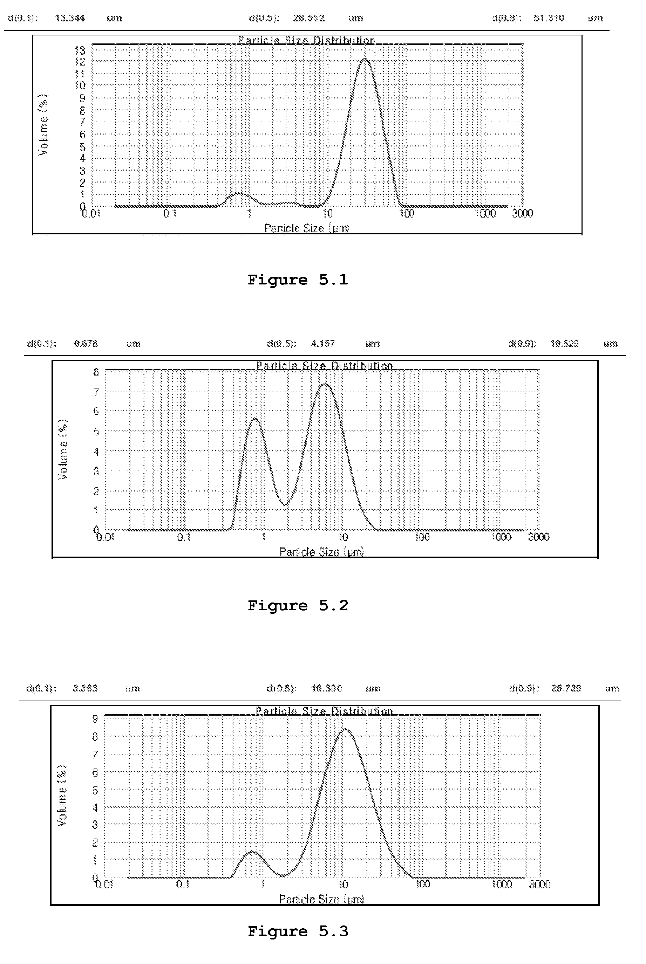

The particle size distribution of hesperidin in the products of the invention is considerably smaller than in commercially available hesperidin as shown in Figures 2 and 5. Commercially available hesperidin has indeed a much broader particle size distribution with the consequence that hesperidin is not stable in solutions and sedimentation occurs rapidly, forming a brown sediment, thus altering the homogeneity and organoleptic properties of the product.

The present inventors have found that by incorporating hesperidin treated according to the present invention, a much more stable product can be obtained. This is exemplified in figure 4, which shows that in a reconstituted milk having commercially available hesperidin particles (variant 1) dark brownish colour sediment forms already after 5 minutes of standing after reconstitution, whereas a reconstituted powder product containing hesperidin according to the invention (variant 2) maintains a "milk-like" colour and is indicative of good stability.

The organoleptic properties of the products of the invention are thereby improved. Thus, liquid products can be obtained which are stable over a period of time and do not alter due to hesperidin sedimentation.

Preferably, the food products of the invention comprise from 0.05-5 wt% hesperidin.

The product according to the present invention may be for example a food product, a drink, a food supplement, a nutraceutical, a cosmetic product, a pet food product or a medicament. While the products are primarily intended to be used by humans, they may also be applied to animals, in particular companion animals, pets or livestock.

Particularly preferred products are products selected from the group consisting of skimmed milk, partially skimmed milk, filled milk, fruit juice, sugar solution, yogurt, sweetened or unsweetened condensed milk, juices, coffee or cereal beverage, chocolate beverage, nutritional oral supplement, gels or liquid supplements for sport, powders such as milk powder, infant formula, dehydrated soup, powdered shake, etc.

In an embodiment, the food products of the invention are in the powder form, preferably selected from milk powder, dehydrated soup powder, shake powder, infant formulae etc. wherein the amount of flavanone is 0.5-5%, preferably 0.5-3%, more preferably 0.5-1%. The powders present the advantage that upon reconstitution in a liquid, the product remains stable for considerable amounts of time compared to products wherein the hesperidin particle size is broader. These powders may be easily reconstituted in a liquid such as water, juice, sugar solution, etc. Typically, 20-30 g of the powder may be mixed with 200-250mL of liquid to produce a drink according to the invention.

In another embodiment, the products of the invention may be any liquid or semi-liquid food product such as skimmed milk, partially skimmed milk, full cream milk, filled milk, yogurt, infant formulae, fruit drink, soup, shake, sweetened or unsweetened condensed milk, juices, coffee or cereal beverages, chocolate beverage, nutritional oral supplement, gels or liquid supplements for sport, etc. and comprise flavanones in an amount of 0.05-0.5%, preferably 0.05-0.3%, more preferably 0.05-0.1%.

The liquid or semi-liquid products may also result from the reconstitution of the powders of the invention with a liquid.

The food products may be water and/or fat-based.

The food products may comprise further ingredients such as fat, vitamins, minerals, probiotics, prebiotics, aromas, colorants, other functional ingredients etc. These may be incorporated at any stage during the processes of the invention.

According to the invention, the food compositions may be used for the manufacture of a composition for the improvement of bone and skin health.

The present invention thus presents the advantage that the processes for the manufacture of food products are simple and help control and reduce the hesperidin particle size. Stable and homogeneous liquid or semi-liquid products obtainable directly according to the invention or reconstituted from the powders of the invention ensue which provides a greater scope of application for hesperidin containing food products.

The present invention is illustrated below by means of non-limiting examples.

### Examples

### Example 1:

A comparative example shown in the table below shows the impact of alkali treatment on hesperidin (variant 2) compared to hesperidin not having undergone the alkali treatment of the invention (variant 1).

**Table 1**

| **Variants** | **1** | **2** |
|---|---|---|
| description | water with 0.1 % of commercially available hesperidin | water with 0.1 % of commercially available hesperidin alkali treated |
| preparation | 0.1g of hesperidin in 99.9g water | 0.1g of hesperidin in 99.9g water. A mix of KOH/NaOH is added to reach pH12. The mix is further acidified to pH 7 using phosphoric acid |
| visual results | brown sediment appears at the bottom of glass after few minutes (2-3 minutes) | opaque suspension |
| % of cumulative distribution of hesperidin particle size below 20 microns | Average 40% | 82% |

Figures 2.2 shows the influence of the alkaline treatment on hesperidin particle size (variant 2) compared to the hesperidin particle size of commercial hesperidin in water (variant 1) (Figure 2.1).

The raw data from which the particle size distribution (PSD) graphs (fig2.1, 2.2) have been extracted, show that for variant 1 (raw hesperidin) only 40% of the cumulative distribution of hesperidin particles are below 20 microns whereas for the alkaline-treated hesperidin it increases to 82% of cumulative distribution of particles below 20 microns.

Figure 3 also shows the different conformation of the hesperidin particles in sample of variant 1 (RM) and in sample of variant 2.

Thus, it can be seen that the alkaline treatment of the invention has an impact on the particle size distribution and the morphology of the hesperidin particles hence on the stability of hesperidin in solution.

### Example 2

An example of a skimmed milk powder formulation containing 1% hesperidin is shown below:

**Table 2 (MSK: skimmed milk solids)**

| | **Skimmed milk powder formulation containing 1% hesperidin** |
|---|---|
| Ingredients | Batch load (kg) |
| Hesperidin solution 5% | 21.31 |
| KOH 30% solution | 1.12 |
| MSK (Medium heated) | 100 |
| water | 118 |
| Total solution | 240 |
| | |
| Total after spray drying | 100 |
| water to evaporate | 140 |

The process to produce the skimmed milk powder containing 1% hesperidin is described below:

The skimmed milk powder is dissolved into water at 65°C into a tank and mixed with a high shear mixer. In parallel, the hesperidin powder is introduced into water at 20°C to reach a % dry matter in the solution of 5%. The mix is mixed for 10min with a high shear mixer. A solution of 30% concentrated potassium hydroxide solution is added to the hesperidin mix until the pH has increased to pH 12. The mix is continuously mixed. This pH 12 mix is then introduced into the milk under constant agitation. The milk is then standardised to a pH of 6.6 to 6.9 and to a dry matter of 40%. The mix is further preheated to 85% with plate heat exchangers. It is then further heat treated at 95°C for 5s in order to get a microbiologically safe product. The mix is further homogenised at 180bar, 1st stage and 40 bar 2nd stage. The mix is further transferred to a high pressure pump and further to a spray dryer in order to obtain the final powder with a % dry matter of about 96%.

In order to reconstitute this powder into a liquid, 25g of this powder is dissolved into warm water (40°C) to obtain a final volume of 250ml. The mix is stirred with a kitchen spoon for 1min. It is then left on a glass table in order to be able to measure possible sedimentation occurring over time.

To measure visual sedimentation (observable by any consumer), a picture of the sample is taken every 5min the first 15min than every 15min the first hour and then every 2h the following 12 hours. The results show a distinct reduction in sedimentation compared to reconstituted powder comprising hesperidin which have not undergone the process of the invention.

### Example 3

This example shows the stability results in a reconstituted milk using hesperidin treated according to the present invention as described in example 2 (variant 2) compared to commercially available hesperidin (variant 1).

Figures 4.1 and 4.2 show the stability of variants 1 and 2 after 5 minutes. The pictures clearly show that in sample of variant 1, a brown sediment forms already after 5 minutes after reconstitution, while sample of variant 2 remains stable.

### Example 4

This example compares the stability of hesperidin in reconstituted milk after alkali treatment (according to the invention) compared to commercially available hesperidin (raw material) and to hesperidin wherein the particle size has been reduced by dry milling.

**Table 3**

| **Variants** | **1** | **2** | **3** |
|---|---|---|---|
| description | MSK powder + 1% standard hesperidin reconstituted in water | MSK powder + 1% hesperidin dry milled reconstituted in water | alkali hesperidin solution at 0.1% hesperidin further acidified to pH 4 used to reconstitute MSK |
| % of cumulative distribution < 20 microns of hesperidin in water | Standard hesperidin Average 40% | Dry-milled hesperidin 99% | Alkaline treated hesperidin 82% |
| preparation | Mix 20g MSK with 0.2g hesperidin standard and add 200ml of 40 deg C water. Mix and follow stability | Mix 20g MSK with 0.2g hesperidin dried mill and add 200ml of 40 deg C water. Mix and follow stability | 200ml water containing 0.1% hesperidin brought to pH 12 and further acidified to pH 4.0 heated to 40deg C and mix in 20g MSK. Mix and follow stability |
| Results | Brown sediment appears at the bottom of glass after few minutes and is clearly visible after 5 min. | light brown sediment is visible after 5 minutes | No sediment is visible even after 12h |

Figures 5.1, 5.2 and 5.3 show respectively the particle size distribution of the hesperidin particles in variants 1, 2, and 3.

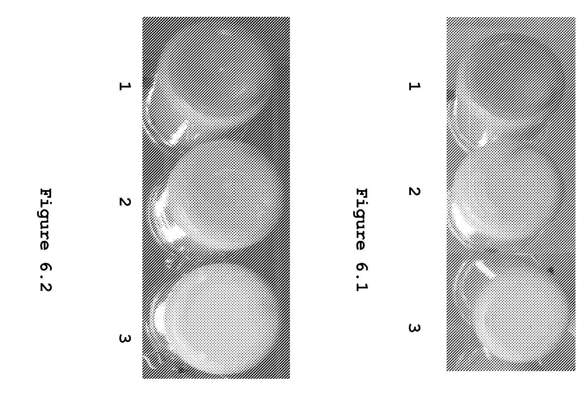

Figures 6.1 and 6.2 show the pictures of variants 1, 2 and 3 taken after 5 minutes and after 12 hours respectively.

These results show that the dry-milling of hesperidin (with 99% of cumulative distribution below 20 microns) leads to a reduction of the sedimentation and gives a lighter sediment colour but does not prevent sedimentation as for hesperidin treated according to the process of the invention at hesperidin concentration of 0.1% in final product. Despite the smaller particle size distribution results of the dry milled hesperidin vs. the alkali treated hesperidin, the stability of hesperidin at 0.1% in liquid milk at or reconstituted powder milk is still better with alkali traeted hesperidin than dry milled hesperidin (with 99% of cumulative distribution below 20 microns).

## Claims

1. Process for the manufacture of a food product comprising flavanones, comprising the step of setting the pH of an aqueous solution of flavanone to a value of 10-14, preferably 12 to 13 prior to mixing the solution with components of the food product.

2. Process according to claim 1, wherein the aqueous solution of flavanone comprises 0.1-30 wt% flavanone, preferably 0.1-20 wt% more preferably about 5 wt%.

3. Process according to claims 1 or 2, wherein the mixture of the aqueous solution comprising flavanone with components of the food product is further processed.

4. Process according to claim 3, wherein the further processing steps comprise any of adjusting the pH, heat treatment, pasteurisation, homogenisation, spray-drying or any combinations thereof.

5. Process according to any of the preceding claims, wherein the food product is any of a powder such as milk powder, dehydrated soup powder, shake powder, infant formulae or a liquid or semi-liquid food product such as skimmed milk, partially skimmed milk, full milk, filled milk, fruit juice, sugar solution, yogurt, sweetened or unsweetened condensed milk, juices, coffee or cereal beverage, chocolate beverage, nutritional oral supplement, soup, shake, gels or liquid supplements for sport

6. Process for improving the stability of flavanones in a liquid or semi-liquid food product comprising the step of setting the pH of an aqueous solution of flavanone to a value of 10-14, preferably 12 to 13 prior to mixing the solution with components of the food product.

7. Process according to any of the preceding claims, wherein the flavanone is hesperidin.

8. Food product obtainable by any of the processes of claim 1 to 5.

9. Food product comprising 0.05-5 wt% flavanones, especially hesperidin, wherein the flavanone is obtained by setting the pH of an aqueous solution of flavanone to a value of 10-14, preferably 12 to 13 prior to mixing the solution with components of the food product.

10. Food product according to claim 9, which is in the powder form, preferably selected from milk powder, dehydrated soup powder, shake powder, infant formulae, and wherein the amount of flavanones is 0.5-5%, preferably 0.5-3%, more preferably 0.5-1%.

11. Food product according to claim 9, which is in the liquid or semi-liquid form, preferably selected from skimmed milk, partially skimmed milk, full milk, filled milk, fruit juice, sugar solution, yogurt, sweetened or unsweetened condensed milk, juices, coffee or cereal beverage, chocolate beverage, nutritional oral supplement, soup, shake, gels or liquid supplements for sport, and wherein the amount of flavanones is 0.05-0.5%, preferably 0.05-0.3%, more preferably 0.05-0.1 %.

12. Food product according to any of claims 8 to 11, wherein the food product is a drink, a food supplement, a nutraceutical, a cosmetic product, a pet food product or a medicament.

13. Use of a food product according to any of claims 8 to 12, for the manufacture of a composition for the improvement of bone and skin health.

14. Process for producing stabilised flavanones comprising the step of setting the pH of an aqueous solution of flavanone to a value of 10-14, preferably 12 to 13.

15. Flavanone obtainable by the process of claim 14.
